(19) Europäisches Patentamt
European Patent Office
Office européen des brevets

(11) EP 0 696 265 B1

(12) **FASCICULE DE BREVET EUROPEEN**

(45) Date de publication et mention de la délivrance du brevet:
**12.05.1999 Bulletin 1999/19**

(21) Numéro de dépôt: **95910576.8**

(22) Date de dépôt: **17.02.1995**

(51) Int Cl.[6]: **C07C 11/02**, C07C 5/27, B01J 21/06
// (B01J21/06, 101:32, 101:50, 101:20)

(86) Numéro de dépôt international:
**PCT/FR95/00190**

(87) Numéro de publication internationale:
**WO 95/23777 (08.09.1995 Gazette 1995/38)**

(54) **PROCEDE POUR L'ISOMERISATION DES OLEFINES**

VERFAHREN ZUR ISOMERISIERUNG VON OLEFINEN

OLEFIN ISOMERISATION METHOD

(84) Etats contractants désignés:
**AT BE DE ES GB IT NL SE**

(30) Priorité: **01.03.1994 FR 9402432**

(43) Date de publication de la demande:
**14.02.1996 Bulletin 1996/07**

(73) Titulaire: **INSTITUT FRANCAIS DU PETROLE**
**92506 Rueil-Malmaison (FR)**

(72) Inventeurs:
- **TRAVERS, Christine**
  **F-92500 Rueil-Malmaison (FR)**
- **BURZYNSKI, Jean-Pierre**
  **F-69110 Sainte-Foy-lès-Lyon (FR)**
- **CHAILLARD, Albert**
  **F-92500 Rueil-Malmaison (FR)**

(56) Documents cités:
**EP-A- 0 588 680** **GB-A- 1 028 168**
**US-A- 2 301 342** **US-A- 2 422 884**

- **DATABASE WPI Week 3484 Derwent Publications Ltd., London, GB; AN 84-210999 & JP,A,59 123 538 (MITSUBISHI PETROCH) , 17 Juillet 1984**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention décrit un procédé d'isomérisation squelettaledes oléfines présentant au plus 20 atomes de carbone et plus particulièrement d'isomérisation des n-butènes en isobutènes et des n-pentènes en isopentènes (iso amylènes), utilisant un catalyseur particulier.

La réduction des alkyls de plomb dans les essences a conduit le raffineur à envisager depuis quelques années, l'incorporation à l'essence de différents composés et en particulier, des alcools et des esters, permettant d'augmenter l'indice d'octane. Outre le méthanol qui est l'un des plus intéressants additifs connu, le MTBE (méthyl-tertio-butyl éther), possède des propriétés antidétonantes permettant l'amélioration de la qualité des essences et l'augmentation de leur indice d'octane, augmentation supérieure à celle obtenue avec le méthanol. Le MTBE possède également de nombreux autres avantages tels que :

- un point d'ébullition correspondant à celui des composants de l'essence qui présentent les plus faibles propriétés antidétonantes,
- une tension de vapeur compatible avec les composants précités,
- un excellent point de congélation,
- une solubilité dans l'eau faible,
- une miscibilité complète avec les hydrocarbures etc.

Le MTBE est généralement obtenu à partir d'isobutène et de méthanol d'après la réaction suivante :

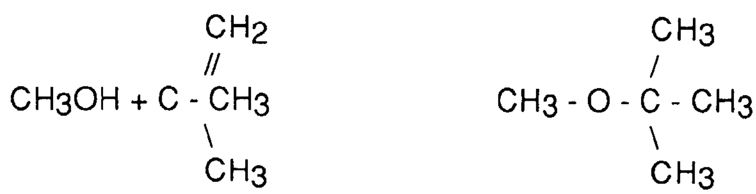

L'isobutène est généralement contenu dans les coupes oléfiniques $C_3$-$C_4$, issues des effluents du craquage catalytique, du vapocraquage, du craquage thermique et du procédé de réduction de viscosité. Cependant les quantités d'isobutène fournies par ces différents procédés ne sont pas suffisantes pour permettre un large développement du procédé de production de MTBE.

C'est pourquoi, il a été proposé, afin de produire de plus grandes quantités d'isobutène, d'isomériser complètement ou presque, les butènes contenus dans les effluents des procédés cités ci-dessus en isobutènes.

De nombreux procédés associés à de nombreux catalyseurs ont été proposés dans la littérature. Les catalyseurs utilisés sont généralement à base d'alumine, ou plus particulièrement d'alumines activées ou traitées à la vapeur (US 3 558 733), qu'il s'agisse d'alumine éta ou gamma, d'alumines halogénées (US 2 417 647), de bauxite, d'alumines traitées avec des dérivés du bore (US 3 558 733), du silicium (US 4 013 590, US 4 038 337, GB 2 129 701 et US 4 434 315) des éléments du groupe III A ou du zirconium et diverses silice-alumines etc.

Le brevet US-2,422,884 décrit un procédé d'isomérisation squelettale en présence d'un catalyseur contenant de l'alumine et de l'oxyde de bore. Une alumine hydratée est décrite avec 8,4% d'eau, 70% d'alumine et également 3,4% $TiO_2$ et 18,2% d'autres éléments (Na, Fe, Si) pour préparer un catalyseur contenant 4,7% $B_2O_3$.

La plupart de ces catalyseurs présente une conversion par passe relativement faible et une faible sélectivité due aux réactions parasites telles que le craquage et la polymérisation, ces dernières entraînant de plus une rapide décroissance des performances, d'où une mauvaise stabilité des performances dans le temps.

### OBJET DE L'INVENTION

Dans un récent brevet de la demanderesse (FR-92/10.949), il a été découvert qu'un catalyseur obtenu à partir d'alumine et de préférence d'alumine éta ou gamma, à laquelle on a ajouté une quantité bien déterminée de titane (0,03 à 0,6 % en poids) et auquel on fait subir de préférence un traitement à la vapeur d'eau dans des conditions précises, conduisait de manière surprenante, à des sélectivités, conversions et durée de cycle très nettement améliorées, si tant est que ce catalyseur est mis en oeuvre en présence de vapeur d'eau.

La présente invention consiste à utiliser, un catalyseur modifié par rapport à celui décrit dans FR-92/10.949, qui présente un rendement en iso-butène accru et surtout une stabilité considérablement plus élevée, et qui permet d'envisager sa mise en oeuvre dans la réaction d'isomérisation squelettale des n-pentènes ou des n-butènes avec

une quantité de vapeur d'eau faible, voire nulle.

Ce catalyseur est obtenu à partir d'alumine et de préférence d'alumine éta ou gamma, à laquelle on ajoute de 0,03 à 0,6 % en poids de titane, et de 0,05 à 5 % en poids d'un oxyde d'un élément du groupe III A (bore, aluminium, gallium, indium, thallium), cet élément étant de manière préférée le bore. On fait ensuite subir de préférence à ce catalyseur, un traitement à la vapeur d'eau, dans des conditions bien précises.

## DISCUSSION DETAILLEE

Dans le procédé suivant l'invention, il est possible d'isomériser soit une coupe $C_4$ oléfinique seule issue des procédés précédemment cités, après avoir enlevé la coupe $C_3$, soit la totalité de la coupe $C_3$-$C_4$ oléfinique.

Selon l'invention, des hydrocarbures oléfiniques linéaires contenant de 5 à 20 atomes de carbone par molécules, peuvent être également isomérisés.

La charge à isomériser est mise au contact du catalyseur à une température comprise entre 300°C et 570°C (et de préférence entre 310°C et 550°C lorsqu'elle est constituée de butènes et/ou de pentènes) à une pression comprise entre 1 et 10 bars absolus (et préférentiellement entre 1 et 5 bars absolus lorsque la charge est constituée de butènes et/ou de pentènes).

La vitesse spatiale est comprise entre 0,1 et 10 $h^{-1}$ exprimée en volume de charge oléfinique par volume de catalyseur et par heure (et de manière préférée entre 0,5 et 6 $h^{-1}$ lorsque la charge est constituée de butènes et/ou de pentènes).

Selon l'invention les réactions secondaires responsables du cokage étant considérablement diminuées le procédé peut être mis en oeuvre en présence de très faibles quantités d'eau, voire même en l'absence d'eau. La quantité d'eau injectée dans le réacteur est telle que le rapport molaire $H_2O$/hydrocarbures oléfiniques est compris entre 0 et 0,3 et de préférence entre 0,05 et 0,25 lorsque la charge est constituée de butènes et ou de pentènes.

Pour la préparation du catalyseur, on peut utiliser des alumines commerciales, de préférence activées, choisies de manière préférée dans le groupe des alumines éta et gamma, et ayant une faible teneur en alcalins, par exemple contenant moins de 0,1 % de sodium.

La surface spécifique de l'alumine sera avantageusement comprise entre 10 et 500 $m^2/g$ et de préférence entre 50 et 450 $m^2/g$, son volume poreux étant compris entre 0,4 et 0,8 $cm^3/g$.

Les catalyseurs selon l'invention, sont préparés en ajoutant de 0,05 à 1 % et de préférence de 0,085 à 0,5 % de bioxyde de titane sur le support d'alumine. Tout procédé permettant l'ajout de ce bioxyde de titane peut convenir. On peut, par exemple, dissoudre un composé de titane dans la solution contenant le composé d'aluminium et ajuster les conditions de précipitation de l'alumine pour que l'hydroxyde de titane coprécipite. On peut également ajouter à l'alumine hydratée sous forme de gel ($\alpha$-trihydrate ou $\beta$-trihydrate, ou $\alpha$-monohydrate d'aluminium) au moins un composé de titane choisi dans le groupe formé par le bioxyde de titane sous les formes rutile et anatase, les sous-oxydes TiO et $Ti_2O_3$, les acides titaniques, les titanates alcalins, alcalinoterreux et d'ammonium et les sels solubles et insolubles, organiques et inorganiques du titane.

On peut également partir d'un support d'alumine mis en forme et l'imprégner avec une solution d'un sel organique ou inorganique de titane ; d'une façon générale, l'addition de titane peut être conduite avant la mise en forme, au cours de la mise en forme ou après mise en forme du support de catalyseur.

Un procédé préféré consiste à ajouter à une solution organique (par exemple alcoolique) d'au moins un composé organique d'aluminium (par exemple un alcoxyaluminium tel que l'isopropylate d'aluminium), au moins un composé organique du titane, par exemple le tétraéthoxytitane, puis à hydrolyser la solution ainsi obtenue.

On peut également ajouter le titane sous forme d'un composé inorganique facilement hydrolysable tel que le tétrachlorure de titane $TiCl_4$.

Un autre procédé préféré consiste à ajouter en quantités contrôlées un composé organique à base de titane, par exemple un alcoxytitane tel que le tétraéthyltitane et/ou un composé inorganique du titane (par exemple le trichlorure de titane) au cours de la synthèse ZIEGLER du polyalcoxyaluminium, par réaction d'un alkylaluminium, (par exemple le triéthylaluminium), de l'éthylène, et d'au moins un composé précité du titane. Par polymérisation puis oxydation subséquente, on prépare le polyalcoxyaluminium précité, dont l'hydrolyse conduira aux polyols et à l'alumine hydratée contenant du titane.

On a constaté expérimentalement que ces procédés conduisaient à une dispersion particulièrement élevée des ions titane dans la matrice d'alumine, obtenue après hydrolyse de l'alcoxyaluminium ou du polyalcoxyaluminium. Les procédés préférés d'imprégnation du titane, permettent, par exemple, lorsque le support se présente sous forme de billes ou d'extrudés etc., d'obtenir une teneur en $TiO_2$ constante d'une bille à l'autre ou d'un extrudé à l'autre ; si la concentration moyenne désirée est C %, la concentration, C, d'une bille à l'autre ou d'un extrudé à l'autre restera, avec les méthodes préférées de l'invention comprise entre C $\pm$ 5 % de cette concentration et même comprise entre $\pm$ 3 % en poids. Des résultats encore améliorés ont été obtenus en utilisant des supports de catalyseur contenant plus particulièrement de 0,06 à 0,15 % de $TiO_2$.

L'oxyde de l'élément du groupe IIIA, peut être déposé indifféremment soit avant soit après le dépôt de titane. Son dépôt peut être réalisé par imprégnation en excès de solution dans une solution acide de l'élément du groupe IIIA considéré. Il peut également être réalisé par imprégnation capillaire diffusionnelle, ou imprégnation à sec, dans le volume poreux du support . Dans le cas du bore, on pourra utiliser l'acide orthoborique, ou tout composé du bore suffisamment soluble.

Le catalyseur ainsi obtenu est ensuite séché à une température comprise entre 100 et 130°C et éventuellement calciné sous air à une température comprise entre 400 et 800°C et de préférence entre 450 et 750°C pendant des temps variant de 1 à 5 heures. Il peut ensuite être avantageusement traité sous vapeur d'eau à une température comprise entre 120 et 700°C et de manière préférée entre 300 et 700°C sous une pression partielle de vapeur d'eau supérieure à 0,5 bar et de préférence comprise entre 0,6 et 1 bar pendant une durée de 0,5 à 120 heures et de préférence de 1 h à 100 h.

Les teneurs en titane et en élément du groupe IIIA sont mesurées par fluorescence X.

Les performances en isomérisation seront exprimées par :

1/ la conversion des butènes

$$C = \frac{\Sigma(\%\ \text{n-butènes})\ \text{charge} - \Sigma(\%\ \text{n-butènes})\ \text{effluent}}{\Sigma\ (\%\text{n-butènes})\ \text{charge}} \times 100$$

2/ la sélectivité en isobutènes

$$S = \frac{(\%\ \text{isobutène})\ \text{effluent} - (\%\text{isobutène})\ \text{charge}}{\Sigma(\%\text{n-butènes})\ \text{charge} - \Sigma(\%\text{n-butènes effluent})} \times 100$$

3/ le rendement en isobutène

$$R = C \times S/100$$

Les exemples qui suivent précisent l'invention sans en limiter la portée.

Tableau 1

| Température (°C) | | 450 | 450 | 470 |
|---|---|---|---|---|
| $H_2O/C_4$=mole | | 0 | 0 | 0,2 |
| LHSV ($h^{-1}$) | | 1 | 1 | 1 |
| Temps de fonctionnement (h) | | 1 | 1 | 1 |
| | Charge | Catalyseur A | Catalyseur B | Catalyseur B |
| $CH_4$ | | 0,06 | 0,06 | 0,05 |
| $C_2$ | | 0,02 | 0,02 | 0,02 |
| $C_2$ = | | 0,15 | 0,12 | 0,09 |
| $C_3$ | | 0,02 | 0,02 | 0,02 |
| $C_3$ = | | 1,7 | 1,2 | 1,05 |
| $iC_4$ | 6,65 | 7,55 | 6,3 | 5,9 |
| $nC_4$ | 20,15 | 21,1 | 22,2 | 22,0 |
| $C_4$ =2TR | 23,7 | 17,35 | 17,43 | 18,17 |
| $C_4$=1 | 32,85 | 12,1 | 11,95 | 11,8 |
| $iC_4$= | 1,3 | 22,4 | 23,90 | 24,0 |
| $C_4$ =2Cis | 15,15 | 13,15 | 13,15 | 13,4 |
| $C_4$= = | 0 | 0 | 0 | 0 |

Tableau 1 (suite)

| | Charge | Catalyseur A | Catalyseur B | Catalyseur B |
|---|---|---|---|---|
| $C_5^+$ | 0,2 | 4,4 | 3,65 | 3,5 |
| Conversion % | | 40,6 | 40,7 | 39,5 |
| Sélectivité % | | 72,7 | 77,4 | 80,1 |
| Rendement % | | 29,5 | 31,5 | 31,65 |

Dans le tableau 1, $C_2$ désigne l'éthane, $C_2$= : l'éthylène, $C_3$ : le propane, $iC_4$ : l'isobutane, $nC_4$ le n-butane, $C_4$=2TR : le butène-2trans, $C_4$=1 : le butène-1, $iC_4$= :l'isobuténe, $C_4$=2Cis : le butène 2Cis, $C_4$== : le butadiène et $C_5^+$ : les hydrocarbures à plus de 5 atomes de carbone.

Tableau 2

| | Catalyseur A | Catalyseur B | Catalyseur B |
|---|---|---|---|
| $H_2O/C_4^=$ (mole) | 0 | 0 | 0,2 |
| LHSV ($h^{-1}$) | 1 | 1 | 1 |
| Température (°c) | 450 | 450 | 470 |
| Temps de fonctionnement (h) | 18 | 18 | 18 |
| Conversion (%) | 36,9 | 39,8 | 39,35 |
| Sélectivité (%) | 73,4 | 77,9 | 80,3 |
| Rendement (%) | 27,1 | 31,0 | 31,6 |

**EXEMPLE 1 Catalyseur A non conforme à l'invention**

Un support d'alumine $\gamma$ commercial de surface 200 $m^2/g$ est imprégné de 0,1 % de titane à partir d'oxalate de titane décahydraté en solution aqueuse, puis séché à 100°C pendant 2 heures et calciné à 600°C pendant 2 heures. Le catalyseur A ainsi obtenu est soumis à un traitement sous vapeur d'eau à 560°C pendant 20 heures avec une pression partielle de vapeur d'eau égale à 0,8 bar. Ce catalyseur est mis en oeuvre en isomérisation d'une coupe $C_4$ oléfinique dont la composition figure tableau 1. Les conditions opératoires sont les suivantes :

| | |
|---|---|
| LHSV = | 1 $h^{-1}$ |
| $H_2O/C_4^=$ (mole) = | 0 |
| T = | 450°C |
| p = | 1 bar absolu |

Les performances obtenues figurent tableau 1 après 1 heure de fonctionnement et tableau 2 après 30 heures de fonctionnement.

**EXEMPLE 2 Catalyseur B conforme à l'invention.**

On dépose sur le catalyseur A préparé dans l'exemple 1 , 1,2 % en poids de bore à partir d'acide borique par imprégnation à sec dans le volume poreux. Le produit obtenu est ensuite séché à 100°C pendant 2 heures, puis calciné à 600°C pendant 2 heures. Le catalyseur B ainsi obtenu est soumis à une traitement sous vapeur d'eau à 560°C pendant 20 heures avec une pression partielle de vapeur d'eau égale à 0,8 bar. Ce catalyseur est mis en oeuvre dans les mêmes conditions que celles décrites dans l'exemple 1. Les performances obtenues figurent tableau 1 après une heure de fonctionnement et tableau 2 après 30 heures de fonctionnement.

Les performances du catalyseur B sont améliorées, notamment en terme de rendement et de stabilité par rapport à celles du catalyseur A.

**EXEMPLE 3 Conforme à l'invention**

Le catalyseur B préparé dans l'exemple 2 est mis en oeuvre en isomérisation d'une coupe $C_4$ oléfinique, dans

des conditions différentes de celles de l'exemple 2, à savoir avec une faible quantité d'eau. Les conditions opératoires sont les suivantes :

| | |
|---|---|
| LHSV = | 1 $h^{-1}$ |
| $H_2O/C_4^=$ (mole) = | 0,2 |
| T = | 470°C |
| p = 1 | bar absolu |

Les performances obtenues figurent tableau 1 après 1 heure de fonctionnement et tableau 2 après 30 heures de fonctionnement. Les performances en terme de rendement et de stabilité sont également dans ces conditions, considérablement améliorées par rapport au catalyseur A.

## Revendications

1. Procédé d'isomérisation squelettale des hydrocarbures oléfiniques linéaires ayant au plus 20 atomes de carbone par molécule, caractérisé en ce que lesdits hydrocarbures sont mis au contact d'un catalyseur contenant de l'alumine et 0,03 à 0,6 % en poids de titane et 0,05 à 5 % en poids d'un oxyde d'un élément du groupe IIIA, à une température comprise entre 300 et 570°C, une pression comprise entre 1 et 10 bars, une vitesse spatiale comprise entre 0,1 et 10 $h^{-1}$.

2. Procédé selon la revendication 1, caractérisé en ce que l'élément du groupe IIIA est le bore.

3. Procédé selon l'une des revendications précédentes, caractérisé en ce que le procédé a lieu en présence d'une injection d'eau telle que le rapport molaire $H_2O$/hydrocarbures oléfiniques est au plus égal à 0,3.

4. Procédé selon l'une des revendications précédentes caractérisé en ce que le catalyseur a subi un traitement à la vapeur d'eau avant d'être mis au contact des hydrocarbures, ledit traitement ayant lieu entre 120 et 700°C sous une pression partielle de vapeur d'eau supérieure à 0,5 bar pendant une durée de 0,5 à 120 h.

5. Procédé selon l'une des revendications précédentes, caractérisé en ce que les hydrocarbures oléfiniques linéaires traités sont choisis dans le groupe formé par les butènes et les pentènes.

6. Procédé selon l'une des revendications précédentes, caractérisé en ce que la température d'isomérisation est comprise entre 310 et 550°C.

7. Procédé selon l'une des revendications précédentes, caractérisé en ce que la pression d'isomérisation est comprise entre 1 et 5 bars.

8. Procédé selon l'une des revendications précédentes, caractérisé en ce que la vitesse spatiale est comprise entre 0,5 et 6 $h^{-1}$.

9. Procédé selon l'une des revendications précédentes, caractérisé en ce que le rapport molaire eau injectée/hydrocarbures est compris entre 0,05 et 0,25.

10. Procédé selon l'une des revendications précédentes, caractérisé en ce que l'alumine est une alumine gamma.

11. Procédé selon l'une des revendications précédentes, caractérisé en ce que l'alumine est une alumine éta.

12. Procédé selon l'une des revendications précédentes, caractérisé en ce que la teneur en sodium de l'alumine est inférieure à 0,1 % en poids.

13. Procédé selon l'une des revendications précédentes, caractérisé en ce que la surface spécifique de l'alumine est comprise entre 10 et 550 $m^2/g$.

14. Procédé selon l'une des revendications précédentes, caractérisé en ce que le volume poreux de l'alumine est compris entre 0,4 et 0,8 $cm^3/g$.

## Patentansprüche

1. Verfahren zur verzweigten Isomerisierung von linearen olefinischen Kohlenwasserstoffen mit höchstens 20 Kohlenstoffatomen pro Molekül, dadurch gekennzeichnet, daß die genannten Kohlenwasserstoffe in Kontakt gebracht werden mit einem Katalysator mit einem Gehalt an Aluminium und 0,03 bis 0.6 Masse-% Titan und 0,05 bis 5 Masse % eines Oxids eines Elements der Gruppe IIIA bei einer Temperatur zwischen 300 und 570° C, einem Druck zwischen 1 und 10 bar und einer Raumgeschwindigkeit zwischen 0,1 und 10 pro Stunde.

2. Verfahren gemäß Anspruch 1, dadurch gekennzeichnet, daß Bor das Element aus Gruppe IIIA ist.

3. Verfahren gemäß einem der vorhergehenden Ansprüche, dadurch gekennzeichnet, daß das Verfahren in Gegenwart einer Wassereinspritzung statt findet, so daß das Molverhältnis $H_2O$/olefinische Kohlenwasserstoffe höchstens gleich 0,3 ist.

4. Verfahren gemäß einem der vorhergehenden Ansprüche, dadurch gekennzeichnet, daß der Katalysator einer Behandlung unter Wasserdampf ausgesetzt war, bevor er mit den Kohlenwasserstoffen in Berührung gebracht wurde, wobei diese Behandlung bei Temperaturen zwischen 120 und 700° C unter einem Partialdruck des Wasserdampfs stattfindet, der höher liegt als 0,5 bar, während einer Dauer von 0,5 bis 120 h.

5. Verfahren gemäß einem der vorhergehenden Ansprüche, dadurch gekennzeichnet, daß die behandelten linearen olefinischen Kohlenwasserstoffe aus der Gruppe ausgewählt sind, die aus Butanen und Pentanen gebildet werden.

6. Verfahren gemäß einem der vorhergehenden Ansprüche, dadurch gekennzeichnet, daß die Isomerisationstemperatur zwischen 310 und 550° C liegt.

7. Verfahren gemäß einem der vorhergehenden Ansprüche, dadurch gekennzeichnet, daß der Isomerisationsdruck zwischen 1 und 5 bar liegt.

8. Verfahren gemäß einem der vorhergehenden Ansprüche, dadurch gekennzeichnet, daß die Raumgeschwindigkeit zwischen 0,5 und 6 pro Stunde liegt.

9. Verfahren gemäß einem der vorhergehenden Ansprüche, dadurch gekennzeichnet, daß das Molverhältnis eingespritztes Wasser/ Kohlenwasserstoffe zwischen 0,05 und 0,25 liegt.

10. Verfahren gemäß einem der vorhergehenden Ansprüche, dadurch gekennzeichnet, daß das Aluminium ein Gamma-Aluminium ist.

11. Verfahren gemäß einem der vorhergehenden Ansprüche, dadurch gekennzeichnet, daß das Aluminium ein Eta-Aluminium ist.

12. Verfahren gemäß einem der vorhergehenden Ansprüche, dadurch gekennzeichnet, daß der Gehalt an Sodium des Aluminiums geringer ist als 0,1 Masse %.

13. Verfahren gemäß einem der vorhergehenden Ansprüche, dadurch gekennzeichnet, daß die spezifische Oberfläche des Aluminiums zwischen 10 und 550 $m^2/g$ liegt.

14. Verfahren gemäß einem der vorhergehenden Ansprüche, dadurch gekennzeichnet, daß das poröse Volumen des Aluminiums zwischen 0,4 und 0,8 $cm^3/g$ liegt.

## Claims

1. A skeletal isomerisation process for linear olefinic hydrocarbons containing at most 20 carbon atoms per molecule, characterised in that said hydrocarbons are brought into contact with a catalyst containing alumina, 0.03% to 0.6% by weight of titanium and 0.05% to 5% by weight of an oxide of an element from group IIIA, at a temperature of between 300°C and 570°C, a pressure of between 1 and 10 bars, and a space velocity of between 0.1 and 10 $h^{-1}$.

2. A process according to claim 1, characterised in that the element from group IIIA is boron.

3. A process according to any one of the preceding claims characterised in that the process takes place in the presence of an injection of water such that the molar ratio of $H_2O$/olefinic hydrocarbons is at most 0.3.

4. A process according to any one of the preceding claims, characterised in that the catalyst undergoes steam treatment before being brought into contact with the hydrocarbons, said treatment taking place at between 120°C and 700°C and at a partial pressure of steam of more than 0.5 bar for a period of 0.5 to 120 h.

5. A process according to any one of the preceding claims, characterised in that the linear olefinic hydrocarbons treated are selected from the group formed by butenes and pentenes.

6. A process according to any one of the preceding claims, characterised in that the isomerisation temperature is between 310°C and 550°C.

7. A process according to any one of the preceding claims, characterised in that the isomerisation pressure is between 1 and 5 bars.

8. A process according to any one of the preceding claims, characterised in that the space velocity is between 0.5 and 6 $h^{-1}$.

9. A process according to any one of the preceding claims, characterised in that the molar ratio of injected water/hydrocarbons is between 0.05 and 0.25.

10. A process according to any one of the preceding claims, characterised in that the alumina is a gamma alumina.

11. A process according to any one of the preceding claims, characterised in that the alumina is an eta alumina.

12. A process according to any one of the preceding claims, characterised in that the sodium concentration in the alumina is less than 0.1% by weight.

13. A process according to any one of the preceding claims, characterised in that the specific surface area of the alumina is between 10 and 550 $m^2/g$.

14. A process according to any one of the preceding claims, characterised in that the pore volume of the alumina is between 0.4 and 0.8 $cm^3/g$.